Europäisches Patentamt

European Patent Office

Office européen des brevets

(11) Veröffentlichungsnummer: **0 153 984**
**B1**

(12) **EUROPÄISCHE PATENTSCHRIFT**

(19)

(45) Veröffentlichungstag der Patentschrift:
30.03.88

(21) Anmeldenummer: **84109692.8**

(22) Anmeldetag: **14.08.84**

(51) Int. Cl.⁴: **C 07 C 7/00**

(54) Verfahren und Vorrichtung zur Gewinnung von C3+-Kohlenwasserstoffen.

(30) Priorität: **09.03.84 DE 3408760**

(43) Veröffentlichungstag der Anmeldung:
**11.09.85 Patentblatt 85/37**

(45) Bekanntmachung des Hinweises auf die Patenterteilung:
**30.03.88 Patentblatt 88/13**

(84) Benannte Vertragsstaaten:
**CH DE FR GB IT LI NL**

(56) Entgegenhaltungen:
**EP - A - 0 010 223**

**TECHNOLOGY, Mai 10, 1982, Seiten 127-137, OIL & GAS
JOURNAL, Tulsa; R.L. ROWELL: "New low-investment
process to recover liquids from refinery fuel gas being
used in Texas"**

(73) Patentinhaber: **Linde Aktiengesellschaft,
Abraham-Lincoln-Strasse 21, D-6200 Wiesbaden (DE)**

(72) Erfinder: **Bauer, Heinz, Dr.rer.nat.Dipl.-Phys.,
Drosselweg 4, D-8027 Neuried (DE)**
Erfinder: **Belloni, Aldo, Dr.-Ing., Rainerstrasse 37,
D-8039 Puchheim (DE)**

(74) Vertreter: **Schaefer, Gerhard, Dr., Linde
Aktiengesellschaft Zentrale Patentabteilung,
D-8023 Höllriegelskreuth (DE)**

## Beschreibung

Die Erfindung betrifft ein Verfahren zur Gewinnung von $C_{3+}$-Kohlenwasserstoffen aus einem Einsatzstrom, der als wesentliche Bestandteile Wasserstoff sowie $C_1$- bis mindestens $C_5$-Kohlenwasserstoffe enthält, bei dem der Einsatzstrom gegebenenfalls vorverdichtet, vorgereinigt und abgekühlt wird, wobei eine flüssige und eine gasförmige Fraktion entspannt wird und die flüssige Fraktion durch Rektifikation in einen im wesentlichen $C_{3+}$-Kohlenwasserstoffe enthaltenden Produktstrom und einen überwiegend $C_{2-}$-Kohlenwasserstoffe enthaltenden Restgasstrom zerlegt wird.

Derartige Verfahren dienen vor allem zur Abtrennung von Propan, gegebenenfalls Propylen und höheren Kohlenwasserstoffen aus Raffinerieabgasen. Die Weiterverarbeitung von Raffinerieabgasen ist in letzter Zeit wirtschaftlich interessant geworden, da die Marktpreise für LPG ($C_3/C_4$-Kohlenwasserstoffgemisch) gestiegen sind, während umgekehrt Vakuumrückstände sowie Schweröl schlecht verkäuflich sind. Aus diesem Grund werden schlecht vermarktbare schwere Produkte zur Deckung des internen Brennstoffbedarfes verfeuert, während gut verkäufliche $C_{3+}$-Kohlenwasserstoffe aus dem Abgas abgetrennt werden.

Es ist bereits ein Verfahren der eingangs genannten Art bekannt geworden (Oil and Gas Journal, May 10, 1982, Seiten 127 bis 131), bei dem ein Raffinerieabgas mit der beschriebenen Zusammensetzung zunächst auf den benötigten Verfahrensdruck verdichtet und, nach Abtrennung von Wasser, gekühlt und teilweise kondensiert wird. Das Kondensat wird einer Trennsäule zugeführt, in der eine flüssige $C_3$-Kohlenwasserstofffraktion und eine gasförmige $C_{2-}$-Kohlenwasserstofffraktion gewonnen werden. Der bei der partiellen Kondensation verbleibende Gasanteil wird in einem Turboexpander entspannt und anschliessend ebenfalls der Trennsäule zugeführt.

Das bekannte Verfahren hat allerdings den Nachteil, dass der gesamte bei der Phasentrennung gewonnene leichte Anteil nach seiner Entspannung zur Erzeugung von Rücklaufflüssigkeit für die Rektifikation in die Trennsäule geleitet wird. Zum einen bedingt die Erhöhung der Konzentration an leichten Bestandteilen in der Trennsäule eine Verschlechterung der Rektifikationsbedingungen. Das bedeutet, dass die Trennsäule bei relativ niedrigen Temperaturen betrieben werden muss und demnach viel Kälteleistung verbraucht. Zum anderen ist der in die Trennsäule eingegebene leichte Anteil ein Zweiphasengemisch. Dies bedeutet wiederum, dass in dem Turboexpander eine Entspannung ins Nassdampfgebiet erfolgt und dass demnach ein hierfür ausgelegter besonders hochwertiger Turboexpander eingesetzt werden muss. Aus den genannten Gründen ist das bekannte Verfahren daher unwirtschaftlich.

Der Erfindung liegt die Aufgabe zugrunde, ein Verfahren der eingangs genannten Art zu entwickeln, das eine Rückgewinnung von $C_{3+}$-Kohlenwasserstoffen mit geringem Investitions- und Betriebsaufwand ermöglicht.

Diese Aufgabe wird erfindungsgemäss dadurch gelöst, dass die gasförmige Fraktion vor der Entspannung, die flüssige Fraktion vor der Rektifikation und die gasförmige Fraktion nach der Entspannung in Wärmetausch mit dem abzukühlenden Einsatzstrom gebracht werden.

Bei dem erfindungsgemässen Verfahren wird die bei der Entspannung erzeugte Kälte nicht wie bisher zur Bildung von Rücklaufflüssigkeit für die Rektifikation, sondern zur Kondensatbildung im Einsatzstrom verwendet. Es ist daher nicht mehr erforderlich, die leichten Anteile aus dem Einsatzstrom wie bisher in die Trennsäule zu leiten. Zugleich entfällt die Notwendigkeit, bei der Entspannung ein zweiphasiges Gemisch zu erzeugen. Dies wird gemäss einem Erfindungsmerkmal dadurch ausgenutzt, dass der zu entspannende Gasstrom vorgewärmt wird, um eine Entspannung ins Nassdampfgebiet zu vermeiden. Das mittels Entspannungskälte erzeugte Kondensat befindet sich auf einem tieferen als dem für die anschliessende Rektifikation benötigten Temperaturniveau. Die überschüssige Kälte wird gemäss einem weiteren Erfindungsmerkmal zur Kühlung des Einsatzstromes verwendet.

Durch die Kombination der erfindungsgemässen Merkmale wird eine Reihe von Vorteilen erreicht, durch die das Verfahren insgesamt wirtschaftlicher wird. Der Verzicht auf die Einleitung der leichten Anteile des Einsatzstromes (insbesondere Wasserstoff sowie $C_1$- und $C_2$-Kohlenwasserstoffe) in die Trennsäule ermöglicht es, die Rektifikation auf einem höheren Temperaturniveau durchzuführen. Das bedeutet, dass bei den üblicherweise vorkommenden Zusammensetzungen des zu rektifizierenden Gemisches einfache und preiswerte Fremdkältekreisläufe im Temperaturbereich oberhalb etwa $-50°C$, typischerweise $-35$ bis $-40°C$, für die Kopfkühlung eingesetzt werden können. Da weiterhin bei der Entspannung keine Kondensatbildung auftritt, lassen sich billigere Entspannungseinrichtungen einsetzen. Schliesslich ergibt die Anwärmung der drei kalten Prozessströme in Wärmetausch mit dem abzukühlenden Einsatzstrom sehr kleine Temperaturdifferenzen am kalten Ende des Wärmetauschers, so dass die Austauschverluste gering sind.

Es erweist sich als vorteilhaft, wenn gemäss einer Weiterbildung des erfindungsgemässen Verfahrens die Abkühlung gegen Fremdkälte und kalte Zerlegungsprodukte erfolgt und die flüssige Fraktion mindestens bis zur Temperatur des kältesten Fremdkälteniveaus und die zu entspannende gasförmige Fraktion weniger stark angewärmt wird. Die gasförmige Fraktion wird vorzugsweise nicht höher als bis zum kältesten Fremdkälteniveau erwärmt.

Bei einer weiteren Ausgestaltung des erfindungsgemässen Verfahrens wird die flüssige Fraktion auf mindestens dasselbe Temperaturniveau wie der bei der Rektifikation gewonnene Gasstrom angewärmt.

Gemäss einer bevorzugten Weiterbildung des erfindungsgemässen Verfahrens wird zumindest ein Teil der gasförmigen Fraktion nach der Entspannung als Regeneriergas bei der Vorreinigung eingesetzt.

Das Gas wird beispielsweise erhitzt und als Spülgas für Adsorber verwendet, die zur Entfernung von Wasser aus dem Einsatzstrom eingesetzt sind.

Es wird weiter vorgeschlagen, dass von der zu entspannenden gasförmigen Fraktion vor der Erwärmung $C_1$- und $C_2$-Kohlenwasserstoffe durch partielle Kondensation abgetrennt werden, um zusätzlich Wasserstoff hoher Reinheit zu erhalten.

Die Abtrennung ist insbesondere dann von Vorteil, wenn der Einsatzstrom einen relativ hohen Wasserstoffanteil aufweist, vorzugsweise in der Grössenordnung von 50 bis 90%. Die Wasserstoffmenge reicht aus, um die benötigte Kälte bei der Entspannung zu erzeugen, so dass der Wasserstoff zurückgewonnen werden kann. Ein Teil des entspannten $H_2$ muss den kondensierten leichten Kohlenwasserstoffen zugegeben werden, um deren Verdampfungsverhalten zu verbessern.

In vielen Anwendungsfällen ist eine weitere Zerlegung des $C_{3+}$-Kohlenwasserstoffproduktes, insbesondere eine Trennung zwischen einem $C_3/C_4$-Kohlenwasserstoffgemisch und $C_{5+}$-Kohlenwasserstoffen erwünscht. Zu diesem Zweck wird gemäss einer bevorzugten Ausgestaltung des erfindungsgemässen Verfahrens vor der Bildung der flüssigen und der gasförmigen Fraktion der Grossteil der $C_{5+}$-Kohlenwasserstoffe zusammen mit einer kleineren Menge von $C_{4-}$-Kohlenwasserstoffen aus dem Einsatzstrom abgetrennt.

Die Abtrennung erfolgt mit Vorteil durch partielle Kondensation bei einer Temperatur, die über der Temperatur liegt, bei der die oben erwähnte flüssige und gasförmige Fraktion gebildet werden. Durch die vorherige Abtrennung ist das der Rektifikation zugeführte Gemisch nahezu frei von $C_{5+}$-Kohlenwasserstoffen. Bei der nachfolgenden Rektifikation wird ein $C_3/C_4$-Kohlenwasserstoff-Produktstrom gewonnen.

Um die Ausbeute von $C_3$- und $C_4$-Kohlenwasserstoffen zu erhöhen, wird weiter vorgeschlagen, dass die abgetrennten schweren Kohlenwasserstoffe ebenfalls der Rektifikation zugeführt werden, dass die Zuführung der schweren Kohlenwasserstoffe unterhalb der Zuführung der flüssigen Fraktion durchgeführt wird und dass ein im wesentlichen $C_3$- und $C_4$-Kohlenwasserstoffe enthaltender Strom zwischen den beiden Zuführungen entnommen wird.

Bei dieser Verfahrensführung werden nicht nur die flüssige Fraktion, sondern auch die vorher aus dem Eingangsstrom abgetrennten schweren Bestandteile rektifiziert. Die Zuführung der beiden Ströme in die Trennsäule erfolgt entsprechend ihrer unterschiedlichen Zusammensetzung an verschiedenen Stellen. Zwischen den beiden Zuführungen befindet sich ein Bereich maximaler $C_3/C_4$-Kohlenwasserstoff-Konzentration. Dort wird ein $C_3/C_4$-Kohlenwasserstoff-Produktstrom entnommen. Zusätzlich wird bei dieser Verfahrensweise ein an $C_{5+}$-Kohlenwasserstoffen reicher Strom gewonnen, der im Sumpf der Trennsäule anfällt und nur wenig $C_4$-Kohlenwasserstoffe enthält.

Bei einer bevorzugten Weiterbildung des erfindungsgemässen Verfahrens wird die gasförmige Fraktion nach ihrer Entspannung in Wärmetausch mit der gasförmigen Phase aus der Phasentrennung des Einsatzstroms bzw. des nach Abtrennung der $C_{5+}$-Kohlenwasserstoffe aus dem Einsatzstrom gebildeten Stromes gebracht.

Durch den Wärmetausch wirkt die für die Phasentrennung eingesetzte Phasentrenneinrichtung als Verstärkungssäule, so dass sich die Ausbeute erhöht. Zusätzliche Betriebskosten fallen nicht an.

Gemäss einer weiteren Ausgestaltung des erfindungsgemässen Verfahrens wird zumindest ein Teil der bei der Entspannung der gasförmigen Fraktion gewonnenen Arbeit zur Verdichtung des Einsatzstromes und/oder zur Nachverdichtung der entspannten gasförmigen Fraktion eingesetzt.

Gemäss einem weiteren Erfindungsmerkmal erfolgt die Phasentrennung des Einsatzstromes bzw. des nach Abtrennung der $C_{5+}$-Kohlenwasserstoffe aus dem Einsatzstrom gebildeten Stromes durch eine Wäsche.

Hierbei ist es insbesondere von Vorteil, wenn gemäss einer weiteren Ausgestaltung des erfindungsgemässen Verfahrens für die Wäsche eine im wesentlichen $C_{5+}$-Kohlenwasserstoffe enthaltende Waschflüssigkeit verwendet wird.

Es wird weiter vorgeschlagen, dass die Waschflüssigkeit aus dem Sumpf einer der Rektifikation nachgeschalteten Zerlegungsstufe entnommen wird.

Eine Vorrichtung zur Durchführung des erfindungsgemässen Verfahrens umfasst eine Zuführungsleitung für einen Einsatzstrom, in der eine Vorreinigungsstufe sowie ein Wärmetauscher mit einer Phasentrenneinrichtung angeordnet sind, deren Flüssigkeitssammelraum mit einer Trennsäule und deren Gassammelraum mit einer Entspannungseinrichtung verbunden sind, und ist dadurch gekennzeichnet, dass die Verbindungen zwischen der Phasentrenneinrichtung und der Rektifiziersäule bzw. der Entspannungseinrichtung sowie eine Abgasleitung der Entspannungseinrichtung durch den Wärmetauscher geführt sind.

Bei einer bevorzugten Weiterbildung der erfindungsgemässen Vorrichtung ist im Gasraum der Phasentrenneinrichtung ein mit dem Ausgang der Entspannungseinrichtung verbundener Wärmetauscher vorgesehen.

Bei einer weiteren Ausgestaltung der erfindungsgemässen Vorrichtung ist als Phasentrenneinrichtung eine Waschsäule vorgesehen.

Es erweist sich als zweckmässig, wenn, wie weiter vorgeschlagen wird, die Entspannungseinrichtung mit einem Verdichter für den Einsatzstrom und/oder mit einem Nachverdichter für das Abgas der Entspannungseinrichtung gekoppelt ist.

Die Erfindung sowie weitere Einzelheiten der Erfindung werden anhand von schematisch dargestellten Ausführungsbeispielen näher erläutert. Hierbei zeigen:

Figur 1 ein Verfahrensschema gemäss der Erfindung,

Figur 2 ein Verfahren mit zusätzlicher Wasserstoff-Rückgewinnung,

Figur 3 und 4 zwei Ausführungsformen eines Verfahrens mit zusätzlicher $C_{5+}$-Abtrennung,

Figur 5 ein Detail einer bevorzugten Ausführungsform des erfindungsgemässen Verfahrens,

Figur 6 ein Verfahrensschema einer weiteren Ausführungsform des erfindungsgemässen Verfahrens.

Bei der Beschreibung der Figuren werden für analoge Bauteile dieselben Bezugszeichen verwendet.

Ein Einsatzstrom 1, der beispielsweise ein Raffinerieabgas ist, wie es bei der Verarbeitung von leichten Rohölbestandteilen zu Benzin anfällt, weist eine Zusammensetzung von 10 bis 90% Wasserstoff, $C_1$ bis $C_5$-Kohlenwasserstoffe, Wasser, Salzsäure sowie gegebenenfalls $C_{6+}$-Kohlenwasserstoffe und Schwefelwasserstoffe auf. Sein Druck beträgt nach einer gegebenenfalls nötigen Vorverdichtung etwa 15 bis 30 bar, seine Temperatur liegt auf Kühlwassertemperaturniveau (25–35°C) oder höher. Der Einsatzstrom wird zunächst durch einen mit Molsieben oder Aluminiumgel gefüllten Reaktor 2 geleitet, in welchem die Salzsäure entfernt wird. Der salzsäurefreie Einsatzstrom wird anschliessend in einem Kühler 3 auf etwa 25–35°C abgekühlt. Dabei kondensiert ein grosser Teil des in dem Einsatzstrom enthaltenen Wassers aus, das in einem Abscheider 4 abgetrennt und über Leitung 5 entfernt wird. Der Einsatzstrom wird anschliessend einem Paar von abwechselnd betriebenen Trocknern 6 zugeführt, die mit Molsieben gefüllt sind. Dort werden letzte Wasserreste aus dem Einsatzstrom entfernt. Der vorgereinigte Einsatzstrom, der die Trockner 6 verlässt, enthält im wesentlichen nur noch Wasserstoff, $C_1$ bis $C_5$-Kohlenwasserstoffe sowie gegebenenfalls $C_{6+}$-Kohlenwasserstoffe und Schwefelwasserstoff. Dieser Strom wird einer gestrichelt dargestellten Coldbox 7 zugeführt, deren wesentliche Bestandteile ein Wärmetauscher 8 sowie eine als Abscheider 9 ausgebildete Phasentrenneinrichtung sind. Die Bauteile 8 und 9 können auch unter Verzicht auf eine Coldbox einzeln isoliert werden. In dem Wärmetauscher 8 wird der vorgereinigte Einsatzstrom 1 in Wärmetausch mit einem Kältemittel, das bei einem (10) oder gegebenenfalls zwei unterschiedlichen (10, 11) Temperaturniveaus verdampft (vorzugsweise −35°C bis −50°C sowie gegebenenfalls zusätzlich 0°C bis −20°C) sowie mit einer Reihe weiterer Prozessströme, die im folgenden noch beschrieben werden, auf etwa −40°C bis −80°C abgekühlt. Bei der Abkühlung kondensiert ein Teil des Einsatzstromes und es bildet sich ein zweiphasiges Gemisch, das dem Abscheider 9 zugeführt wird. Aus dem Abscheider 9 wird eine gasförmige Fraktion 12, die nahezu den gesamten in dem Einsatzstrom enthaltenen Wasserstoff sowie einen Grossteil der $C_1$ und $C_2$-Kohlenwasserstoffe enthält und eine flüssige Fraktion 13, die im wesentlichen aus $C_{3+}$-Kohlenwasserstoffen sowie einem geringen Anteil an $C_1$ und $C_2$-Kohlenwasserstoffen besteht, entnommen. Die gasförmige Fraktion 12 wird durch einen Teil des Wärmetauschers 8 geleitet und dort in Wärmetausch mit dem Einsatzstrom 1 auf eine Temperatur angewärmt, die vorzugsweise nicht höher als die des Kältemittels 10 ist. Sie verlässt den Wärmetauscher 8 an einer Zwischenstelle und wird einer Expansionstrubine 14 zugeführt, in welcher sie auf etwa 5 bis 15 bar entspannt wird. Diese Entspannung kann gegebenenfalls zweistufig mit Zwischenanwärmung erfolgen. Die Anwärmung des zu entspannenden Gasstromes im Wärmetauscher 8 ist so gewählt, dass bei der Entspannung keine Kondensatbildung auftritt. Die Entspannungsturbine ist bei Bedarf mit einem Verdichter für den Einsatzstrom gekoppelt (nicht dargestellt).

Die entspannte gasförmige Fraktion 12, deren Temperatur etwa 3 bis 20°C unter der des Abscheiders 9 liegt, wird durch den Wärmetauscher 8 geleitet und in Wärmetausch mit dem Einsatzstrom 1 angewärmt. Ein Teilstrom 15 des Gasstromes 12 wird als Brenngas aus der Anlage abgezogen, während ein anderer Teilstrom 16, gegebenenfalls nach Erwärmung in einem Erhitzer 17, als Regeneriergas für die im Wechselbetrieb arbeitenden Trockner 6 eingesetzt wird. Nach der Regenerierung wird das beladene Regeneriergas dem Teilstrom 15 zugeführt.

Die flüssige Fraktion 13 aus dem Abscheider 9 wird ebenfalls durch einen Teil des Wärmetauschers 8 geleitet und erwärmt sich in Wärmetausch mit dem abzukühlenden Einsatzstrom 1. Die Erwärmung erfolgt vorzugsweise bis auf das Temperaturniveau des oberen (wärmeren) Kältekreislaufes 11. Nach ihrer Erwärmung wird die flüssige Fraktion 13 an einer Zwischenstelle aus dem Wärmetauscher 8 entnommen und einer Trennsäule 18 zugeführt, in der eine Rektifikation des flüssigen Gemisches durchgeführt wird. Die Trennsäule 18 wird bei einem Druck von etwa 10 bis 20 bar vorzugsweise 13–15 bar betrieben. In ihr wird eine Trennung zwischen der $C_{2-}$ und der $C_{3+}$-Fraktion durchgeführt. Bei der beschriebenen Verfahrensführung ist es ausreichend, den Kopf der Trennsäule 18 auf etwa −35 bis −50°C zu kühlen. Dies geschieht durch Verdampfen eines Fremdkältemittels im Kopfkondensator 19, das dem Kältemittel 10 entspricht. Für Wärmetauscher 8 und Kondensator 19 wird ein gemeinsamer Kältekreislauf verwendet.

Der Sumpf der Trennsäule 18 ist durch einen externen Aufkocher 20 beheizt, der beispielsweise durch Niederdruckdampf oder Heisswasser beheizt ist.

Bei der Rektifikation wird ein dampfförmiges Kopfprodukt 21, das hauptsächlich aus $C_1$ und $C_2$-Kohlenwasserstoffen mit höchstens 1 bis 3% $C_{3+}$-Kohlenwasserstoffen besteht (Restgasstrom) und eine flüssige Sumpffraktion 22, die im wesentlichen $C_{3+}$-Kohlenwasserstoffe und höchstens 1 bis 10% $C_2$-Kohlenwasserstoffe enthält, gewonnen. Der flüssige $C_{3+}$-Produktstrom 22 wird gegebenenfalls in einem Kühler 23 unterkühlt und mittels einer Pumpe 24 auf den benötigten Abgabedruck gebracht. Die gasförmige Fraktion 21 wird auf einen Druck entspannt, der nur um soviel über dem Brenngasdruck liegt, um den Druckverlusten beim Anwärmen und Regenerieren der Adsorber Rechnung zu tragen. Anschliessend wird sie entsprechend ihrer Temperatur an einer Zwischenstelle in den Wärmetauscher 8 eingeführt und, nach Anwärmung gegen den abzukühlenden Einsatzstrom ↑, der entspannten gasförmigen Fraktion 12 zugemischt.

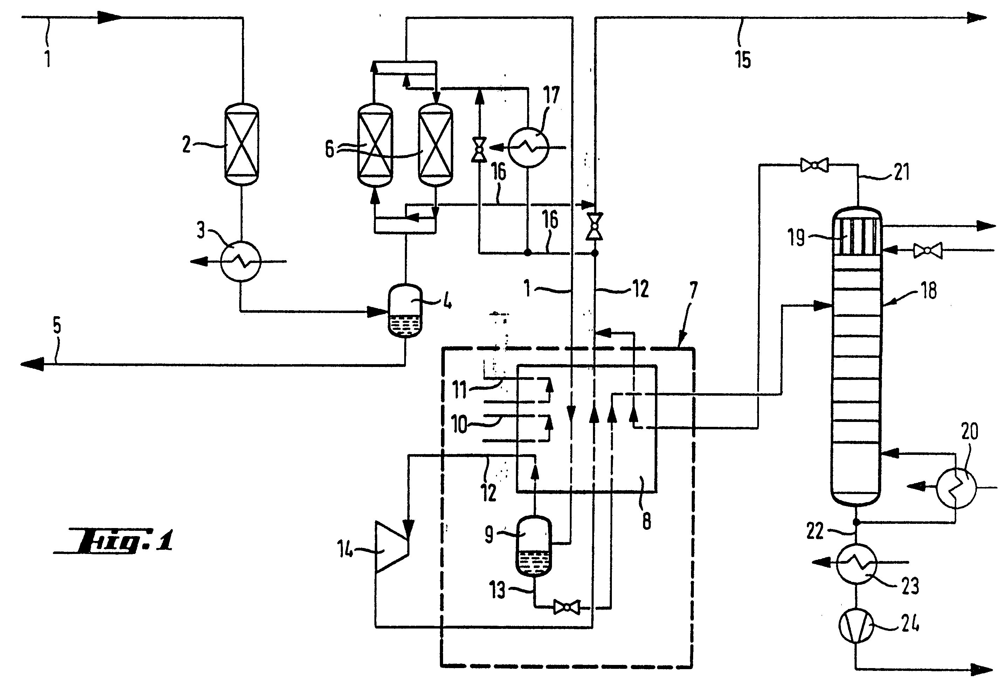

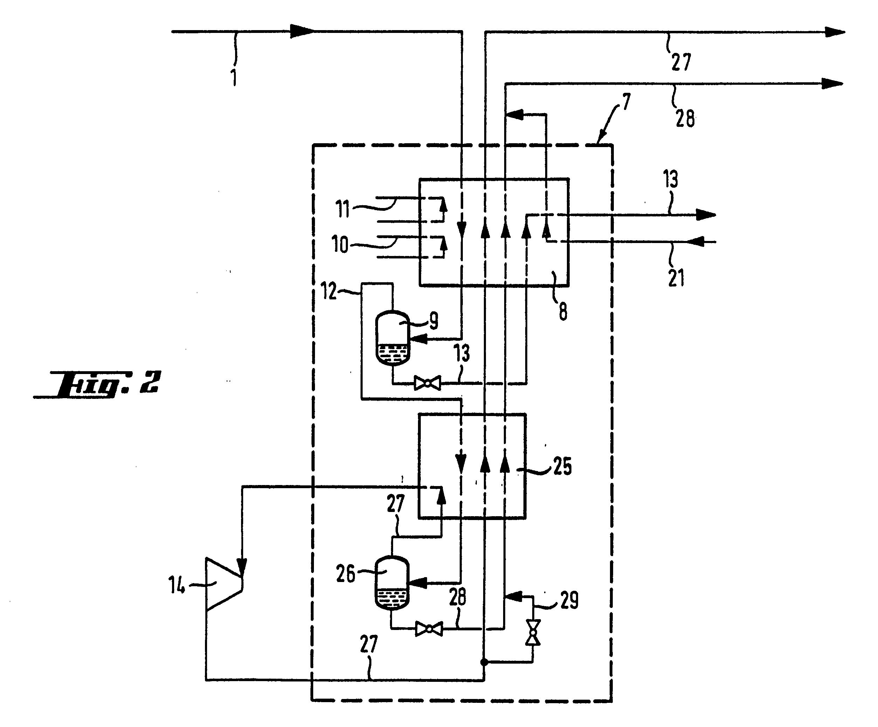

Bei dem Verfahrensschema gemäss Figur 2 sind die Vorreinigungsstufen sowie die Rektifikation nicht dargestellt. Im Gegensatz zu dem anhand von Figur 1 beschriebenen Verfahren wird die gasförmige Fraktion 12 aus dem Abscheider 9 nicht unmittelbar in der Turbine 14 entspannt, sondern zunächst einem Wärmetauscher 25 zugeführt, in welchem sie in Wärmetausch mit drei kalten Strömen, die im folgenden noch beschrieben werden, abgekühlt wird. Am kalten Ende des Wärmetauschers 25 besitzt die Fraktion 12 eine Temperatur von ca. $-140$ bis $-170°C$. Bei dieser Temperatur kondensiert ein Teil der in der Fraktion 12 enthaltenen Komponenten. In einem Abscheider 26 wird das Zweiphasengemisch in eine gasförmige Phase 27, die im wesentlichen aus Wasserstoff besteht, und eine flüssige Phase 28, die im wesentlichen $C_1$ und $C_2$-Kohlenwasserstoffe enthält, getrennt. Die gasförmige Phase 27 wird durch einen Teil des Wärmetauschers 25 geleitet und in Wärmetausch mit dem abzukühlenden Strom 12 angewärmt, bevor sie der Turbine 14 zugeführt wird. In der Turbine 14 wird der Gasstrom 27 auf etwa 5 bis 15 bar entspannt. Die Vorwärmung des zu entspannenden Stromes 27 dient dazu, eine Kondensatbildung bei der Entspannung zu vermeiden.

Der entspannte Gasstrom 27 wird nacheinander durch die Wärmetauscher 25 und 8 geführt, wobei er sich in Wärmetausch mit den abzukühlenden Strömen 12 bzw. 1 erwärmt.

Die flüssige Phase 28 wird ebenfalls nacheinander durch die Wärmetauscher 25 und 8 geleitet und erwärmt. Zwischen den Leitungen 27 und 28 befindet sich vor Eintritt in den Wärmetauscher 25 eine Verbindungsleitung 29, durch welche dem $C_1/C_2$-Kohlenwasserstoffgemisch 28 eine gewisse Menge Wasserstoff 29 zugemischt wird, um den Partialdruck der Kohlenwasserstoffe abzusenken und eine Verdampfung bei niederer Temperatur zu ermöglichen.

Das Verfahren gemäss Figur 2 bietet die Möglichkeit, neben einer $C_{3+}$-Kohlenwasserstofffraktion Wasserstoff als Produkt zu gewinnen. Dieses Verfahren ist wirtschaftlich nur dann sinnvoll, wenn der im Einsatzstrom 1 enthaltene Wasserstoffanteil ausreichend ist, um in der Turbine 14 die benötigte Kälte zu erzeugen.

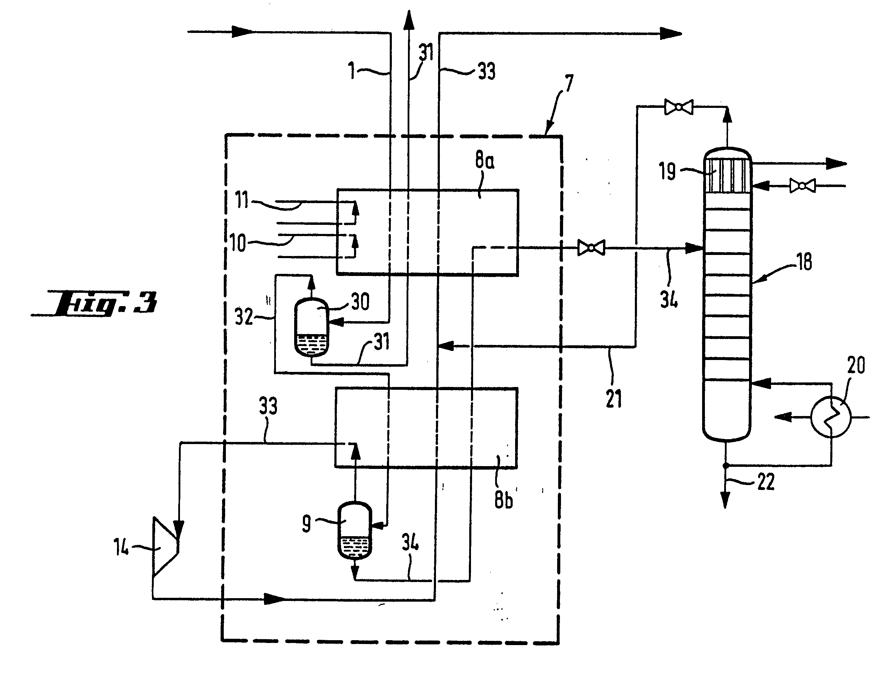

Der in Figur 3 dargestellte Ausschnitt des Verfahrensschema gemäss Figur 1 zeigt eine Ausführungsform des erfindungsgemässen Verfahrens, bei der als Produkt ein $C_3/C_4$-Kohlenwasserstoffgemisch ohne $C_{5+}$-Anteil gewonnen wird. Bei diesem Verfahren erfolgt die Abkühlung des Einsatzstromes 1 in zwei Stufen, nämlich in zwei Wärmetauscherblöcken 8a und 8b. Je nach Grösse der Blöcke können diese auch in einem zusammenhängenden Stück gebaut werden. Nach Abkühlung im Wärmetauscher 8a gegen Fremdkältemittel, wobei dessen höheres Verdampfungsniveau (11) oder das niedrigere (10) oder beide zusammen (10, 11) in Abhängigkeit von Rohgaszusammensetzung und Druck eingesetzt werden, sowie drei im folgenden noch beschriebenen Strömen 31, 33, 34 besitzt der Einsatzstrom 1 eine Temperatur von etwa $-10$ bis $-35°C$. Bei dieser Temperatur kondensiert der grösste Teil der $C_{5+}$-Kohlenwasserstoffe aus. Das Zweiphasengemisch wird einem Abscheider 30 zugeführt, in welchem die gasförmige von der flüssigen Phase getrennt wird. Die flüssige Phase, die im wesentlichen $C_{5+}$-Kohlenwasserstoffe sowie $C_3$ und $C_4$-Kohlenwasserstoffe enthält, wird über Leitung 31 entnommen und durch den Wärmetauscher 8a geleitet, in welchem sie gegen den Einsatzstrom 1 angewärmt wird.

Die gasförmige Phase, die im wesentlichen Wasserstoff sowie $C_1$ bis $C_4$-Kohlenwasserstoffe enthält, wird über Leitung 32 entnommen und im Wärmetauscher 8b weiter abgekühlt. Dabei entsteht ein zweiphasiges Gemisch, das dem Abscheider 9 zugeführt wird. Die gasförmige Phase 33, die im wesentlichen Wasserstoff sowie $C_1$ und $C_2$-Kohlenwasserstoffe enthält, wird nach Anwärmung im Wärmetauscher 8b der Turbine 14 zugeführt, in welcher sie entspannt wird. Die Erwärmung im Wärmetauscher 8b wird soweit durchgeführt, dass bei der Entspannung keine Kondensatbildung auftritt.

Der entspannte Gasstrom 33 wird durch die Wärmetauscher 8b und 8a geleitet, in welchen er gegen den Einsatzstrom 1 bzw. den Strom 32 angewärmt wird.

Die flüssige Fraktion 34 aus dem Abscheider 9, die im wesentlichen $C_3$ und $C_4$-Kohlenwasserstoffe sowie $C_1$ und $C_2$-Kohlenwasserstoffe enthält, wird im Wärmetauscher 8b angewärmt und im Wärmetauscher 8a bis etwa auf das Temperaturniveau des Kältemittels 10 weiter erwärmt. Der Strom wird an einer Zwischenstelle aus dem Wärmetauscher 8a entnommen und in die Trennsäule 18 eingeführt. Dort findet die anhand Figur 1 bereits beschriebene Rektifikation statt mit dem Unterschied, dass das Sumpfprodukt 22 nahezu frei von $C_{5+}$-Kohlenwasserstoffen ist.

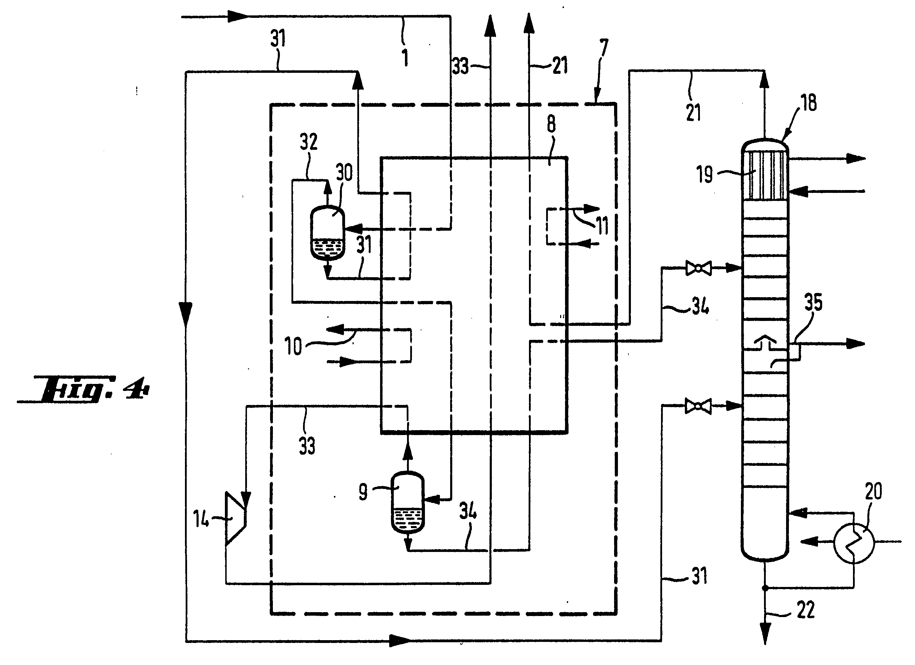

Das Verfahren gemäss Figur 3 hat allerdings den Nachteil, dass bei der vorab vorgenommenen Abtrennung von $C_{5+}$-Kohlenwasserstoffen im Abscheider 30 ein nicht unbeträchtlicher Teil von $C_3$ und $C_4$-Kohlenwasserstoffen mit dem Kondensat abgetrennt werden. Eine höhere Ausbeute an $C_3$ und $C_4$-Kohlenwasserstoffen ermöglicht eine Ausführungsform des Verfahrens, die anhand Figur 4 beschrieben wird.

Der getrocknete Einsatzstrom 1 wird analog zu dem in Figur 3 dargestellten Verfahren nur bis zu einer Temperatur abgekühlt, bei dem die $C_{5+}$-Kohlenwasserstoffe kondensieren. Das an einer Zwischenstelle aus dem Wärmetauscher 8 entnommene Zweiphasengemisch wird dem Abscheider 30 zugeführt, in dem eine Phasentrennung durchgeführt wird. Die gasförmige Phase 32 wird an einer Zwischenstelle in den Wärmetauscher 8 zurückgeführt und am kalten Ende daraus entnommen. Die flüssige Phase 31 wird dagegen nach Anwärmung in einem Teil des Wärmetauschers 8 der Trennsäule 18 zugeführt. Die Zuführung erfolgt unterhalb der Stelle, an der die Fraktion 34 aus dem Abscheider 9 in die Trennsäule 18 eingeführt wird.

Die Trennsäule 18 besitzt eine grössere Anzahl von Böden als die Trennsäule bei dem Verfahren gemäss Figur 3. Sie weist zwischen den beiden Zuführungen der Ströme 31 und 34 eine Entnahmeleitung 35 auf. In diesem Bereich befindet sich eine maximale Konzentration an $C_3$ und $C_4$-Kohlenwasserstoffen.

Im Sumpf der Trennsäule 18 wird über Leitung 22 eine Flüssigkeit entnommen, die im wesentlichen $C_{5+}$-Kohlenwasserstoffe enthält. Vom Kopf der Trennsäule 18 wird über Leitung 21 eine Gasfraktion entnommen, die im wesentlichen $C_1$ und $C_2$-Kohlenwasserstoffe enthält.

Im Gegensatz zu dem Verfahren gemäss Figur 3 werden bei diesem Verfahren die schweren Anteile, die im Abscheider 30 abgetrennt worden sind, ebenfalls der Rektifikation zugeführt. Auf diese Weise lässt sich mit relativ geringem Aufwand eine sehr hohe Ausbeute an $C_3$ und $C_4$-Kohlenwasserstoffen erzielen.

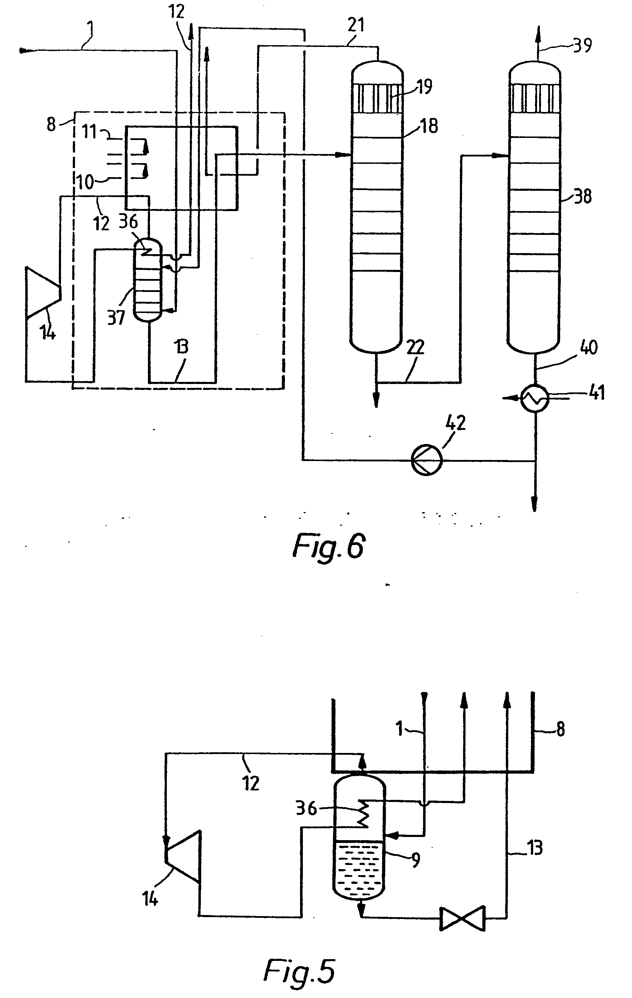

Bei dem in Figur 5 dargestellten Verfahrensdetail ist im Gasraum des Abscheiders 9 ein Wärmetauscher 36 vorgesehen, der an die Abgasseite der Turbine 14 angeschlossen ist. Durch den Wärmetauscher 36 arbeitet der Abscheider 9 wie eine Verstärkungssäule, so dass sich die Ausbeute bei der Phasentrennung erhöht. Der Wärmetauscher 36 kann sowohl bei der Verfahrensführung gemäss Figur 1, bei der der Einsatzstrom 1 in den Abscheider 9 eingeleitet wird, als auch bei der Verfahrensführung gemäss Figur 3 oder Figur 4, bei der ein nach Abtrennung der $C_{5+}$-Kohlenwasserstoffe aus dem Einsatzstrom gebildeter Strom 32 in den Abscheider 9 eingeleitet wird, vorgesehen sein.

Bei dem Verfahren gemäss Figur 6 wird die Phasentrennung des Einsatzstromes 1 in einer Waschsäule 37 durchgeführt. Die über Kopf abziehende gasförmige Fraktion 12 wird in der Turbine 14 arbeitsleistend entspannt, der entspannte Gasstrom wird in einem Wärmetauscher 36 zur Kühlung des Kopfes der Waschsäule 37 eingesetzt. Die flüssige Sumpffraktion aus der Waschsäule 37 wird über Leitung 13 der Rektifiziersäule 18 zugeführt. Die $C_{3+}$-Fraktion 22, die aus dem Sumpf der Rektifikationssäule 18 entnommen wird, wird einer weiteren Rektifikationssäule 38 zugeführt, in der eine Trennung in eine $C_3/C_4$-Fraktion, die über Leitung 39 vom Kopf der Rektifikationssäule 38 entnommen wird und eine $C_{5+}$-Fraktion, die über eine Leitung 40 aus dem Sumpf der Rektifiziersäule 38 entnommen wird, erfolgt. Die $C_5$-Fraktion wird in einem Kühler 41 gekühlt und mittels einer Pumpe 42 als Rücklauf auf die Waschsäule 37 aufgegeben.

**Patentansprüche**

1. Verfahren zur Gewinnung von $C_{3+}$-Kohlenwasserstoffen aus einem Einsatzstrom, der als wesentliche Bestandteile Wasserstoff sowie $C_1$- bis mindestens $C_5$-Kohlenwasserstoffe enthält, bei dem der Einsatzstrom gegebenenfalls vorverdichtet, vorgereinigt, abgekühlt, teilweise kondensiert und in eine flüssige und eine gasförmige Fraktion getrennt wird, und bei dem die gasförmige Fraktion entspannt wird und die flüssige Fraktion durch Rektifikation in einen im wesentlichen $C_{3+}$-Kohlenwasserstoffe enthaltenden Produktstrom und einen überwiegend $C_{2-}$-Kohlenwasserstoffe enthaltenden Restgasstrom zerlegt wird, dadurch gekennzeichnet, dass die gasförmige Fraktion vor der Entspannung, die flüssige Fraktion vor der Rektifikation und die gasförmige Fraktion nach der Entspannung jeweils in Wärmetausch mit dem abzukühlenden Einsatzstrom gebracht werden.

2. Verfahren nach Anspruch 1, dadurch gekennzeichnet, dass die Abkühlung gegen Fremdkälte und kalte Zerlegungsprodukte erfolgt und die flüssige Fraktion mindestens bis zur Temperatur des kältesten Fremdkälteniveaus und die zu entspannende gasförmige Fraktion weniger stark angewärmt wird.

3. Verfahren nach Anspruch 1 oder 2, dadurch gekennzeichnet, dass die flüssige Fraktion auf mindestens dasselbe Temperaturniveau wie der bei der Rektifikation gewonnene Restgasstrom angewärmt wird.

4. Verfahren nach einem der Ansprüche 1 bis 3, dadurch gekennzeichnet, dass zumindest ein Teil der gasförmigen Fraktion nach der Entspannung als Regeneriergas bei der Vorreinigung eingesetzt wird.

5. Verfahren nach einem der Ansprüche 1 bis 4, dadurch gekennzeichnet, dass von der zur entspannenden gasförmigen Fraktion vor der Erwärmung $C_1$- und $C_2$-Kohlenwasserstoffe durch partielle Kondensation abgetrennt werden.

6. Verfahren nach einem der Ansprüche 1 bis 5, dadurch gekennzeichnet, dass vor der Bildung der flüssigen und der gasförmigen Fraktion der Grossteil der $C_{5+}$-Kohlenwasserstoffe aus dem Einsatzstrom abgetrennt wird.

7. Verfahren nach Anspruch 6, dadurch gekennzeichnet, dass die abgetrennten schweren Kohlenwasserstoffe ebenfalls der Rektifikation zugeführt werden, dass die Zuführung der $C_{5+}$-Kohlenwasserstoffe unterhalb der Zuführung der flüssigen Fraktion durchgeführt wird und dass ein im wesentlichen $C_3$- und $C_4$-Kohlenwasserstoffe enthaltender Strom zwischen den beiden Zuführungen entnommen wird.

8. Verfahren nach einem der Ansprüche 1 bis 7, dadurch gekennzeichnet, dass die gasförmige Fraktion nach ihrer Entspannung in Wärmetausch mit der gasförmigen Phase aus der Phasentrennung des Einsatzstromes bzw. des nach Abtrennung der $C_{5+}$-Kohlenwasserstoffe aus dem Einsatzstrom gebildeten Stromes gebracht wird.

9. Verfahren nach einem der Ansprüche 1 bis 8, dadurch gekennzeichnet, dass zumindest ein Teil der bei der Entspannung der gasförmigen Fraktion gewonnenen Arbeit zur Verdichtung des Einsatzstromes und/oder zur Nachverdichtung der entspannten gasförmigen Fraktion eingesetzt wird.

10. Verfahren nach einem der Ansprüche 1 bis 9, dadurch gekennzeichnet, dass die Phasentrennung des Einsatzstromes bzw. des nach Abtrennung der $C_{5+}$-Kohlenwasserstoffe aus dem Einsatzstrom gebildeten Stromes durch eine Wäsche erfolgt.

11. Verfahren nach Anspruch 10, dadurch gekennzeichet, dass für die Wäsche eine im wesentlichen $C_{5+}$-Kohlenwasserstoffe enthaltende Waschflüssigkeit verwendet wird.

12. Verfahren nach Anspruch 11, dadurch gekennzeichnet, dass die Waschflüssigkeit aus dem Sumpf einer der Rektifikation nachgeschalteten Zerlegungsstufe entnommen wird.

13. Vorrichtung zur Durchführung des Verfahrens nach Anspruch 1 mit einer Zuführungsleitung für einen Einsatzstrom, in der eine Vorreinigungsstufe sowie ein Wärmetauscher mit einer Phasentrenneinrichtung angeordnet sind, deren Flüssigkeitssammelraum mit einer Trennsäule und deren Gassammelraum mit einer Entspannungseinrichtung verbunden sind, dadurch gekennzeichnet, dass die Verbindungen zwischen der Phasentrenneinrichtung (9) und der Rektifiziersäule (18) bzw. der Entspannungseinrichtung (14) sowie eine Abgasleitung (15, 27, 33) der Entspannungseinrichtung (14) durch den Wärmetauscher (8) geführt sind.

14. Vorrichtung nach Anspruch 13, dadurch gekennzeichnet, dass im Gasraum der Phasentrenneinrichtung (9) ein mit dem Ausgang der Entspannungseinrichtung (14) verbundener Wärmetauscher (36) vorgesehen ist.

15. Vorrichtung nach Anspruch 12 oder 13, dadurch gekennzeichnet, dass als Phasentrenneinrichtung (9) eine Waschsäule (37) vorgesehen ist.

16. Vorrichtung nach einem der Ansprüche 13 bis 15, dadurch gekennzeichnet, dass die Entspannungseinrichtung (14) mit einem Verdichter für den Einsatzstrom (1) und/oder mit einem Nachverdichter für das Abgas der Entspannungseinrichtung (14) gekoppelt ist.

## Claims

1. Process for obtaining $C_{3+}$-hydrocarbons from an input stream which contains as essential components hydrogen and $C_1$- to at least $C_5$-hydrocarbons, wherein the input stream is, if necessary, pre-compressed, pre-purified, cooled partially condensed and separated into a liquid and a gaseous fraction, and wherein the gaseous fraction is expanded and the liquid fraction is separated by rectification into a product stream essentially containing $C_{3+}$-hydrocarbons and a residual gas stream containing preponderantly $C_2$-hydrocarbons, characterised in that the gaseous fraction before expansion, the liquid fraction before rectification and the gaseous fraction after expansion are all brought into heat exchange with the input stream to be cooled.

2. A process as claimed in Claim 1, characterised in that the cooling is effected against external cold and cold separation products, and the liquid fraction is heated at least to the temperature of the coldest level of external cold and the gaseous fraction to be expanded is heated less strongly.

3. A process as claimed in Claim 1 or 2, characterised in that the liquid fraction is heated to at least the same temperature level as the residual gas stream obtained by the rectification.

4. A process as claimed in one of Claims 1 to 3, characterised in that at least a part of the gaseous fraction after expansion is introduced as regeneration gas for the pre-purification.

5. A process as claimed in one of Claims 1 to 4, characterised in that $C_1$- and $C_2$-hydrocarbons are separated by partial condensation from the gaseous fraction to be expanded before the heating step.

6. A process as claimed in one of Claims 1 to 5, characterised in that the greater part of the $C_{5+}$-hydrocarbons are separated from the input stream before the formation of the liquid and gaseous fractions.

7. A process as claimed in Claim 6, characterised in that the separated heavy hydrocarbons are also fed to the rectification step; that the introduction of the $C_{5+}$-hydrocarbons is effected below the introduction of the liquid fraction; and that a stream containing essentially $C_3$- and $C_4$-hydrocarbons is withdrawn between the two points of introduction.

8. A process as claimed in one of Claims 1 to 7, characterised in that after its expansion, the gaseous fraction is brought into heat exchange with the gaseous phase resulting from the phase separation of the input stream, or of the stream formed after separation of the $C_{5+}$-hydrocarbons from the input stram, as the case may be.

9. A process as claimed in any of Claim 1 to 8, characterised in that at least a part of the work produced by the expansion of the gaseous fraction is applied to the compression of the inlet stream and/or to the re-compression of the expanded gaseous fraction.

10. A process as claimed in one of Claims 1 to 9, characterised in that the phase separation of the input stream, or of the stream formed after the separation of the $C_{5+}$-hydrocarbons from the input stream, as the case may be, results from a washing step.

11. A process as claimed in Claim 10, characterised in that a washing liquid containing essentially $C_{5+}$-hydrocarbons is used for the washing step.

12. A process as claimed in Claim 11, characterised in that the washing liquid is withdrawn from the sump of a separation stage used for the rectification.

13. Apparatus for carrying out the process as claimed in Claim 1, comprising an inlet line for an input stream, in which is arranged a pre-purification stage, as well as a heat exchanger with a phase-separation device whose liquid collecting space is connected to a separation column and whose gas separation space is connected to an expansion device, characterised in that the connections between the phase-separation device (9) and the rectification column (18) and the expansion device (14), as well as an exhaust gas line (15, 27, 33), are led through the heat exchanger (8).

14. Apparatus as claimed in Claim 13, characterised in that a heat exchanger (36) connected to the outlet of the expansion device (14) is provided in the gas space of the phase separation device (9).

15. Apparatus as claimed in Claim 12 or 13, characterised in that a washing column (37) is provided as phase-separation device (9).

16. Apparatus as claimed in one of Claims 13 to 15, characterised in that the expansion device (14) is coupled to a compressor for the input stream (1) and /or to a re-compressor for the exhaust gas from the expansion device (14).

**Revendication**

1. Procédé d'extraction d'hydrocarbures en $C_{3+}$ à partir d'une charge d'alimentation essentiellement formée d'hydrogène ainsi que d'hydrocarbures en $C_1$ à $C_5$ au moins, dans lequel ladite charge est éventuellement précomprimée, prépurifiée, refroidie, partiellement condensée et séparée en une phase liquide et une phase gazeuse, la phase gazeuse est détendue et la phase liquide est séparée par rectification en un produit final gazeux contenant essentiellement les hydrocarbures $C_{3+}$ et en un courant gazeux résiduaire contenant principalement des hydrocarbures $C_{2-}$, caractérisé en ce que la phase gazeuse avant détente , la phase liquide avant rectification et la phase gazeuse après détente font chacune l'objet d'échange de chaleur avec ladite charge d'alimentation à refroidir.

2. Procédé selon la revendication 1, caractérisé en ce que le refroidissement est assuré par apport frigorique externe et par échange thermique avec des produits de fractionnement froids, et en ce que la phase liquide est réchauffée à une au moins jusqu'à la température du plus bas apport frigorifique externe, cependant que la phase gazeuse à détendre est réchauffée à un degré moindre.

3. Procédé selon l'une des revendication 1 ou 2, caractérisé en ce que la phase liquide est réchauffée au moins au même niveau de température que le courant gazeux résiduaire provenant de la rectification.

4. Procédé selon l'une des revendications 1 à 3, caractérisé en ce qu'au moins une partie de la phase gazeuse est utilisée, après la détente, en tant que gaz de régénération lors de la prépurification.

5. Procédé selon l'une des revendications 1 à 4, caractérisé en ce que les hydrocarbures en $C_1$ et $C_2$ sont séparés par condensation partielle de la phase gazeuse à détendre, préalablement au réchauffage.

6. Procédé selon l'une des revendications 1 à 5, caractérisé en ce qu'avant la formation des phases liquide et gazeuse, la majeure partie des hydrocarbures en $C_{5+}$ est séparée de la charge d'alimentation.

7. Procédé selon la revendication 6, caractérisé en ce que les hydrocarbures lourds séparés sont également amenés à la rectification, en ce que les hydrocarbures en $C_{5+}$ sont amenés à un niveau inférieur à celui auquel est amené la phase liquide et en ce qu'un courant essentiellement formé des hydrocarbures en $C_3$ et $C_4$ est retiré entre ces deux niveaux respectifs.

8. Procédé selon l'une des revendications 1 à 7, caractérisé en ce que la phase gazeuse est soumise, après détente, à un échange de chaleur avec la phase gazeuse obtenue lors de la séparation des phases de la charge d'alimentation ou avec le courant formé après séparation des hydrocarbures en $C_{5+}$ de ladite charge d'alimentation.

9. Procédé selon l'une des revendications 1 à 8, caractérisé en ce qu'au moins une partie du travail fourni lors de la détente de la phase gazeuse est utilisé pour comprimer la charge d'alimentation et/ou pour post-comprimer la phase gazeuse détendue.

10. Procédé selon l'une des revendications 1 à 9, caractérisé en ce que la séparation de phases de la charge d'alimentation ou du courant formé après séparation des hydrocarbures en $C_{5+}$ de ladite charge est effectuée par lavage.

11. Procédé selon la revendication 10, caractérisé en ce qu'on utilise pour le lavage un liquide de lavage contenant essentiellement des hydrocarbures en $C_{5+}$.

12. Procédé selon la revendication 11, caractérisé en ce que le liquide de lavage provient de la cuve d'une zone de fractionnement disposée en aval de la zone de rectification.

13. Dispositif pour la mise en œuvre du procédé selon la revendication 1, comportant une conduite d'amenée d'une charge d'alimentation, une zone de prélavage ainsi qu'un échangeur de chaleur avec une appareillage de séparation de phases dont l'enceinte collectrice de liquide est reliée à une colonne de rectification, cependant que son enceinte collectrice de gaz est reliée à un détendeur, caractérisé en ce que les connexions entre l'appareillage de séparation de phases (9) et la colonne de rectification (18) ou le détendeur (14), ainsi qu'une conduite de sortie de gaz (15, 27, 33) provenant du détendeur (14) traversent l'échangeur de chaleur (8).

14. Dispositif selon la revendication 13, caractérisé en ce que l'enceinte collectrice de gaz de l'appareillage de séparation de phases (9) renferme un échangeur de chaleur raccordé à la sortie du détendeur (14).

15. Dispositif selon la revendication 12 ou 13, caractérisé en ce que l'appareillage de séparation de phases est constitué par une colonne de lavage.

16. Dispositif selon l'une des revendications 13 à 15, caractérisé en ce que le détendeur (14) est couplé à un compresseur pour la charge d'alimentation (1) et/ou à un post-compresseur pour le gaz sortant du détendeur.

**Fig.1**

Fig. 2

0153984

**Fig. 3**

Fig. 4

0153984

Fig.6

Fig.5

17